Europäisches Patentamt

European Patent Office

Office européen des brevets

(11) Veröffentlichungsnummer: **0 200 672**
**A1**

## (12) EUROPÄISCHE PATENTANMELDUNG

(21) Anmeldenummer: 86730063.4

(22) Anmeldetag: 04.04.86

(51) Int. Cl.⁴: **A 61 F 2/36**

(30) Priorität: 04.04.85 DE 8510531 U

(43) Veröffentlichungstag der Anmeldung:
05.11.86 Patentblatt 86/45

(84) Benannte Vertragsstaaten:
CH DE FR GB LI

(71) Anmelder: **MECRON MEDIZINISCHE PRODUKTE GMBH**
**Nunsdorfer Ring 23-27**
**D-1000 Berlin 48(DE)**

(72) Erfinder: **Ahrens, Uwe**
**Nürnberger Strasse 46**
**D-1000 Berlin 30(DE)**

(74) Vertreter: **Christiansen, Henning, Dipl.-Ing.**
**Dietrich-Schäfer-Weg 21**
**D-1000 Berlin 41(DE)**

(54) Implantat.

(57) Implantat mit einem Konusanschluß mit Mitteln zum Lösen eines auf den Außenkonus aufgesteckten Elements, insbesondere einer Gelenkkugel, wobei der Außenkonus (20) mit einer Längsbohrung (3) versehen ist, welche in eine quer gerichtete Öffnung (6) mündet, die sich seitlich bis zur Außenoberfläche erstreckt und eine dem Konusanschlußzugewandte Anlagefläche (21) aufweist, und in der Längsbohrung (3) verschieblicher Stift (5) vorgesehen ist, der sich, ausgehend von der Frontfläche des Konus (20), bis in die quer gerichtete Öffnung (6) hinein erstreckt.

Fig. 2    Fig. 1

Croydon Printing Company Ltd.

EP 0 200 672 A1

MECRON                                      2. April 1986
medizinische Produkte GmbH

D-1000 Berlin

ME35.2-EU

---

## Implantat

---

## B e s c h r e i b u n g

Die Erfindung betrifft ein Implantat der im Oberbegriff
des Anspruchs 1 angegebenen Art.

Bei der Behandlung von durch Trauma oder Krankheit schwer
funktionsgeschädigten Gelenken des Menschen hat sich die Endoprothese allgemein durchgesetzt. In den letzten Jahren werden immer häufiger Endoprothesen mit einem Konus versehen, auf dem man verschiedene Steckköpfe aufsetzen kann. Diese Konussteckverbindung führt schon bei kleinsten Aufsetzkräften zu einer hochfesten Verbindung. Dabei tauchen vor allem zwei Probleme auf:

1. Nach Aufsetzen der Kugel und Reponieren des Gelenks wird intraoperativ häufig festgestellt, daß eine andere Halslänge eingestellt werden muß. Dazu ist es nötig, die bereits aufgesetzte Kugel abzuziehen.

2. Bei Reoperationen ist es oftmals nicht notwendig, den Prothesenstiel zu wechseln. Jedoch ist es aus tribologischen Gesichtspunkten heraus vernünftig, die Kugel durch eine ungeschädigte zu ersetzen.

Die derzeit auf dem Markt befindlichen Kugelabziehgeräte sind jeweils nur für einen Teilbereich der Implantate verwendbar und ermöglichen in den meisten Fällen kein für den Schaft biegemomentfreies Lösen der Steckkugel.

Ein Implantat der eingangs genannten Gattung ist aus der DE-OS 33 19 916 bekannt. Bei der bekannten Lösung wird die Gelenkkugel mittels einer Mutter gelöst, welche unterhalb der Gelenkkugel auf einem dort befindlichen Gewindeansatz aufgeschraubt ist, wenn die Mutter in Richtung auf die Gelenkkugel mittels eines Schlüssels "abgeschraubt" wird.

Diese Lösung hat den Nachteil, daß durch die direkt unterhalb der Gelenkkugel vorgesehene Mutter in manchen Fällen die Bewegungsfähigkeit des Gelenkes beschränkt ist bzw. der Schaftdurchmesser in seinen äußeren - für die Festigkeit besonders wichtigen - Bereichen geschwächt wird.

Der Erfindung liegt die Aufgabe zugrunde, ein Implantat mit Mitteln zum Lösen der Gelenkkugel vom Konusanschluß zu schaffen, das mit einfachen Werkzeugen betätigt werden kann und die Außenabmessungen von Schaft- und Kugel nicht beeinflußt.

Es wurde ein Kugellösemechanismus entwickelt, der im Prothesenhals integriert ist und für sämtliche derzeit verwendeten Kugeltypen eine einwandfreie und für den Prothesenstiel biegemomentfreie Ablösung des Kopfes erlaubt.

Dazu ist in den Prothesenhals eine Bohrung parallel zur Längsachse eingebracht. Des weiteren ist der Prothesenhals im von der aufgesetzten Kugel nicht abgedeckten Bereich mit einem Langloch versehen, das parallel zur Längsachse und die Zentralbohrung treffend verläuft. In die zentrale Bohrung ist ein abgesetzter Stift eingesetzt, der durch eine Sicke im oberen Bereich am Herausfallen gehindert wird.

Das Lösen eines aufgebrachten Steckkopfes erfolgt bevorzugt mittels eines Keiles, der in das Langloch mit einem geeigneten Instrument eingedrückt wird.

Besonders vorteilhaft bei der erfindungsgemäßen Lösung ist, daß die konstruktiv einfache Lösung die äußere Mechanik und wirksame Kontur der Verbindung Gelenkkugel/Schaft nicht beeinflußt, da der die Gelenkkugel gegebenenfalls lösende Stift sich zentral im Inneren des Konus befindet, der die Gelenkkugel trägt.

Bei günstigen Weiterbildungen der Erfindung ist der Stift zum Inneren hin geringfügig erweitert oder gekrümmt, so daß er bei abgenommener Gelenkkugel nicht herausfällt, sich aber durch den den Außenkonus tragenden Ansatz für die Gelenkkugel eintreiben läßt.

Bei einer anderen vorteilhaften Weiterbildung der Erfindung ist anstelle eines quer durch den Hals des Schaftes zu treibenden Keils eine in einer Querbohrung vorgesehene Schraube angebracht, deren Schaft einen nicht mit einem Gewinde versehenen Bereich aufweist, der sich entweder konusförmig erweitert oder aber exzentrisch ausgebildet ist. Auf diese Weise kann mit einem herkömmlichen (vorzugsweise Inbus-) Schlüssel eine Drehung erzeugt werden, welche den Stift innerhalb der im Hals der Prothese vorgesehenen Längsbohrung nach oben treibt, so daß die Gelenkkugel abgehoben wird.

Es ist ersichtlich, daß die Wege, welche zum Lösen der Gelenkkugel vom Stift durchmessen werden müssen, äußerst kurz sind, so daß der auf den Stift zu übertragende Hub ebenfalls nur klein zu sein braucht. Somit ist auch bei einer in einer Querbohrung einzubringenden Schraube nur ein Konus mit einem relativ kleinen Steigungsverhältnis bzw. mit einer geringen Exzentrizität erforderlich, vorausgesetzt daß die Konuspassung mit der notwendigen Genauigkeit hergestellt ist und somit die Gelenkkugel einen definierten Sitz hat.

Die erfindungsgemäßen Mittel sind in gleicher Weise für Bausätze von Prothesen verwendbar, welche mittels Konusverbindungen in unterschiedlichen Längen zusammensteckbar sind, wobei also auch der Prothesenschaft aus mittels Konusverbindungen steckbaren Einzelteilen bestehen kann.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1    ein erstes Ausführungsbeispiel der Erfindung in Seitenansicht,

Figur 2    dasselbe Ausführungsbeispiel im Schnitt,

Figur 3    ein Detail einer weiteren Ausführung der Erfindung im Schnitt sowie

Figuren 4 und 5 Details des weiteren Ausführungsbeispiels in einer zur Darstellungsebene der Figur 3 um 90° gedrehten Schnittebene.

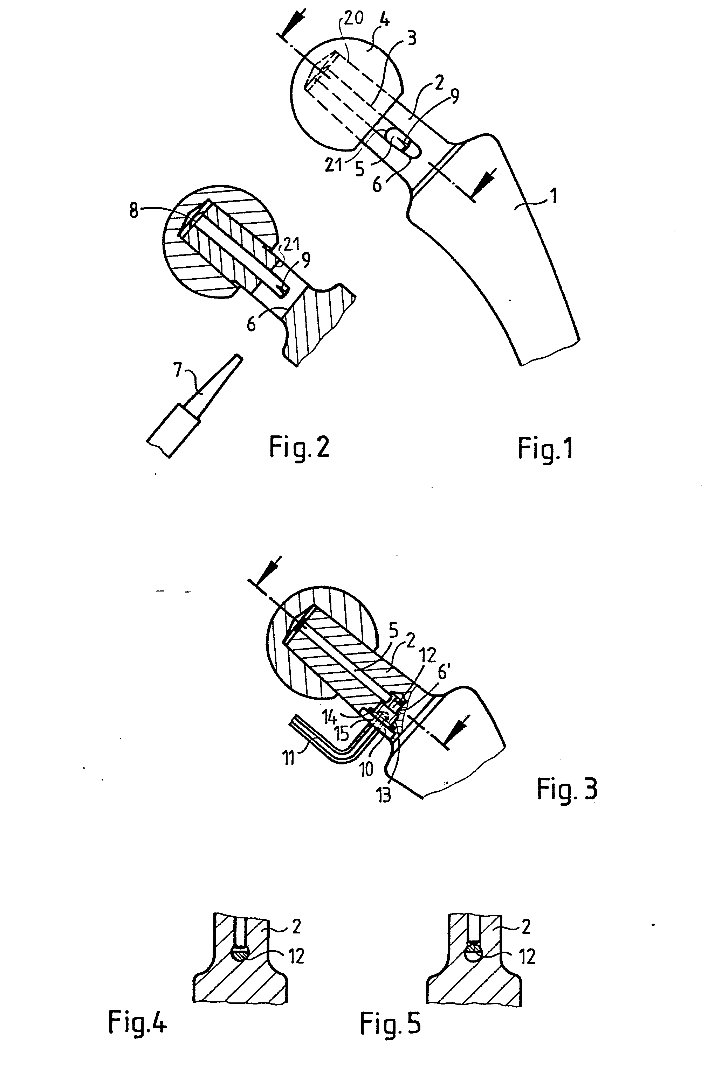

Im Figur 1 ist eine Hüftgelenkprothese 1 wiedergegeben, welche einen Hals 2 aufweist, der mit einer Längsbohrung 3 versehen ist, welche koaxial im Hals verläuft und in Form einer Sackbohrung zum eine Gelenkkugel 4 tragenden Ende des Halses 2 hin offen ist. Innerhalb der Bohrung 3 ist ein in Längsrichtung verschieblicher Stift 5 vorgesehen, welcher im unteren - von der Gelenkkugel 4 freien - Teil des Halses 2 mittels einer Queröffnung 6 erreichbar ist.

Die innerhalb des Außenkonus vorgesehene Längsbohrung 3, die durch die Stirnfläche des am Hals 2 befindlichen Konus 20 hindurchtritt, ist derart ausgebildet, daß das entgegengesetzte Ende der Bohrung in die Queröffnung 6 mündet, die sich bis zur Außenoberfläche des Halses erstreckt und eine der Frontfläche zugewandte Anlagefläche (21) für einen Keil oder dergleichen aufweist. Diese Anlagefläche wird in der Regel durch die der Konusverbindung zugewandte Begrenzungsfläche der Öffnung 6 gebildet.

Der in der Längsbohrung verschiebliche Stift 5 erstreckt sich, ausgehend von der Stirnfläche des Konus - bis in die Queröffnung 6 hinein. Letztere ist bei Verwendung eines Keils vorzugsweise durchgehend ausgebildet.

Die Queröffnung 6 weist in Richtung des Durchmessers der Bohrung 3 einen geringfügig stärkeren Querschnitt auf, so daß der in der Öffnung 3 vorgesehene Stift 5 nicht vollständig zum Grund der Queröffnung herunter bewegbar ist, und der untere Teil der Queröffnung 6 somit zur Einführung eines Keils 7 offen bleibt. Die Größenverhältnisse sind anhand der Schnittdarstellung gemäß Figur 2 erkennbar.

Eine andere Möglichkeit, den Stift 5 am Herunterfallen zu hindern, besteht darin, an seinem der Frontfläche der Konus zugewandten Ende eine geringfügige Erweiterung 8 nach Art eines Nietkopfes vorzusehen. Auf diese Weise kann die Gelenkkugel - falls es bei einer Reoperation erforderlich ist - jederzeit schnell und unproblematisch mittels des Keils 7, auf den der Schlag ausgeübt wird, entfernt werden.

Der Stift 5 weist an seinem unteren Ende eine geringfügige Erweiterung 9 auf, welche derart bemessen ist, daß der Stift 5 daran gehindert ist, bei abgenommener Gelenkkugel 4 aus der Öffnung 3 herauszufallen. Diese Erweiterung 9 ist durch einen Körnerschlag oder mittels einem entsprechenden scharfkantigen Instrument durch die Öffnung 6 hindurch erzeugt worden, als der Stift 5 bereits eingesetzt war, so daß der Halt noch sicherer ist.

Bei dem in Figur 3 dargestellten Detail eines weiteren Ausführungsbeispiels ist zum Entfernen der Gelenkkugel kein Schlag erforderlich, sondern es kann der Stift 5 mittels einer Schraube 10 über einen (gestrichelt dargestellten) Inbus-Schlüssel 11 betätigt werden. Die Schraube 10 ist mittels eines Gewindes 13 in die Queröffnung 6' eingesetzt und weist einen Exzenter 12 auf, an den sich das Ende des Stiftes 5 anlegt. Je nach Stellung des Exzenters 12 wird der Stift 5 nun mehr oder weniger weit in Richtung der Gelenkkugel herausgeschoben, wobei die Exzenterposition von außen her durch eine Markierung an der Schraube ablesbar ist. Der Schraubenkopf weist eine Dichtung 14 auf, welche das Eindringen von Körperflüssigkeit in das Innere des Halses 2 verhindert, so daß die Funktion nicht durch Verdickung oder Gerinnung behindert werden kann. Außerdem ist in dem Schraubenkopf eine mehrkantige Ausnehmung zur Aufnahme des Schlüssels 11 vorgesehen.

In den Figuren 4 und 5 ist der Exzenter 12 in zwei verschiedenen Positionen wiedergegeben, wobei ersichtlich ist, daß in Figur 5 der Stift 5 zum Entfernen der Gelenkkugel 4 angehoben ist.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen. Dabei sind insbesondere verschiedene Vorrichtungen zum Betätigen des Stiftes 5 als Alternativen günstig. Neben einem Keil kann ein rundes in eine entsprechend gestaltete Aussparung einführbares Instrument mit einem konischen oder exzentrischen Schaft verwendet werden. Ist die Aussparung mit einem Gewinde 13 versehen, so brauchen die Vorschub- oder Haltekräfte des Betätigungsinstruments nicht von außen her aufgebracht zu werden. Sie werden vielmehr direkt in den Prothesenschaft eingeleitet. Eine einfache Realisierungsmöglichkeit bildet dabei die dargestellte Schraube 10, die entweder im Implantat verbleibt oder aber ein separates bedarfsweise einzusetzendes Instrument bildet.

* * * * *

Ansprüche

1. Implantat mit einem Konusanschluß mit Mitteln zum Lösen eines auf den Außenkonus aufgesteckten Elements, insbesondere einer Gelenkkugel,

d a d u r c h   g e k e n n z e i c h n e t ,

daß der Außenkonus (20) mit einer Längsbohrung (3) versehen ist, welche in eine quer gerichtete Öffnung (6) mündet, die sich seitlich bis zur Außenoberfläche erstreckt und eine dem Konusanschluß zugewandte Anlagefläche (21) aufweist, und daß

ein in der Längsbohrung (3) verschieblicher Stift (5) vorgesehen ist, der sich, ausgehend von der Frontfläche des Konus (20), bis in die quer gerichtete Öffnung (6) hinein erstreckt.

2. Implantat nach Anspruch 1, d a d u r c h   g e -k e n n z e i c h n e t ,   daß die sich in Querrichtung erstreckende Öffnung (6) durch den den Konus (20) aufweisenden Hals (2) hindurch erstreckt.

3. Implantat nach einem der vorangehenden Ansprüche, d a d u r c h   g e k e n n z e i c h n e t ,   daß die sich in Querrichtung erstreckende Öffnung (6) in Längsrichtung eine größere Abmessung aufweist als in der zu Längs- und Querrichtung senkrecht stehenden dritten Raumrichtung.

/.

4. Implantat nach einem der vorangehenden Ansprüche, d a d u r c h   g e k e n n z e i c h n e t ,   daß der Stift (5) leicht gekrümmt verläuft und/oder eine Erweiterung (9) an seinem dem Konus (20) abgewandten Ende aufweist, die den Durchmesser der sich in Längsrichtung erstreckenden Öffnung (3) überragt.

5. Implantat nach einem der vorangehenden Ansprüche, d a d u r c h   g e k e n n z e i c h n e t ,   daß der Stift (5) eine Erweiterung (8) an seinem dem Konusanschluß zugewandten Ende aufweist, die den Durchmesser der sich in Längsrichtung erstreckenden Öffnung (3) überragt.

6. . Implantat nach einem der Ansprüche 4 oder 5, d a - d u r c h   g e k e n n z e i c h n e t ,   daß es sich bei der Erweiterung (8, 9) um eine Anquetschung handelt.

7. Implantat nach einem der vorangehenden Ansprüche, d a d u r c h   g e k e n n z e i c h n e t ,   daß die sich in Querrichtung erstreckende Öffnung (6') ein Gewinde (13) aufweist, in das eine Schraube (10) einfügbar ist.

8. Implantat nach Anspruch 7, d a d u r c h   g e - k e n n z e i c h n e t ,   daß eine in die Öffnung (6') eingefügte Schraube (10) im Bereich ihres Schaftes mit einem konusförmigem und/oder exzentrischen Ansatz (12) versehen ist.

9.    Implantat nach einem der Ansprüche 7 oder 8, d a -
d u r c h   g e k e n n z e i c h n e t ,  daß der Kopf der
Schraube (10) einen Innensechskant (15) aufweist.

* * * * *

/

0200672.

Fig. 2            Fig. 1

Fig. 3

Fig. 4            Fig. 5

# EUROPÄISCHER RECHERCHENBERICHT

**0200672**

Nummer der Anmeldung

EP 86 73 0063

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| D,Y | DE-A-3 319 916 (MECRON MEDIZINISCHE PRODUKTE GMBH) * Anspruch 1; Figur 1 * | 1 | A 61 F 2/36 |
| Y | AT-B- 284 588 (H. BILZ WERKZEUGFABRIK) * Anspruch 1; Figuren 1, 2 * | 1 | |
| A | * Figuren 1, 2; Seite 2, Zeilen 23 - 29 * | 2,4 | |
| A | DE-C- 854 739 (COLLET & ENGELHARD) * Figur 1 * | 7-9 | |
| A | GB-A-1 371 335 (NATIONAL RESEARCH DEVELOPMENT CORP.) * Figur; Seite 1, Zeilen 83-89 * | 1 | RECHERCHIERTE SACHGEBIETE (Int. Cl.4) A 61 F 2/00 B 23 B 31/00 |

Der vorliegende Recherchenbericht wurde für alle Patentansprüche erstellt

| Recherchenort BERLIN | Abschlußdatum der Recherche 25-06-1986 | Prüfer KANAL P K |
|---|---|---|

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82